(19) Europäisches Patentamt
European Patent Office
Office européen des brevets

(11) Veröffentlichungsnummer: **0 365 478 B1**

(12) **EUROPÄISCHE PATENTSCHRIFT**

(45) Veröffentlichungstag der Patentschrift: **08.12.93**

(51) Int. Cl.5: **C09B 62/503**, C07C 317/04, D06P 1/384

(21) Anmeldenummer: **89810770.1**

(22) Anmeldetag: **10.10.89**

(54) **Reaktivfarbstoffe, deren Herstellung und Verwendung.**

(30) Priorität: **18.10.88 CH 3877/88**

(43) Veröffentlichungstag der Anmeldung:
**25.04.90 Patentblatt 90/17**

(45) Bekanntmachung des Hinweises auf die Patenterteilung:
**08.12.93 Patentblatt 93/49**

(84) Benannte Vertragsstaaten:
**BE CH DE FR GB IT LI**

(56) Entgegenhaltungen:
**EP-A- 0 210 951**
**EP-A- 0 221 013**

(73) Patentinhaber: **CIBA-GEIGY AG**
**Klybeckstrasse 141**
**CH-4002 Basel(CH)**

(72) Erfinder: **Tzikas, Athanassios**
**Unt. Rütschetenweg 36**
**CH-4133 Pratteln(CH)**
Erfinder: **Aeschlimann, Peter**
**Sandweg 16**
**CH-4123 Allschwil(CH)**

**EP 0 365 478 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Reaktivfarbstoffe werden seit langem in grossem Umfang für das Färben und Bedrucken von Textilien aus Fasermaterialien eingesetzt, und es steht heute eine grosse Anzahl von brauchbaren Reaktivfarbstoffen mit unterschiedlichen Eigenschaften und für verschiedene Anwendungsbereiche zur Verfügung. Angesichts der immer höheren Anforderungen an Reaktivfärbungen in bezug auf Wirtschaftlichkeit, Applikationstechnik und Echtheitsniveau ist der erreichte technische Stand aber vielfach nicht voll befriedigend.

So ist z.B. häufig festzustellen, dass der Fixiergrad zu gering, und die Differenz zwischen Ausziehgrad und Fixiergrad zu gross ist (hoher Seifverlust), so dass ein erheblicher Teil des Reaktivfarbstoffes für den Färbevorgang verloren geht. Ferner lässt das Aufbauvermögen in vielen Fällen zu wünschen übrig.

Der vorliegenden Erfindung lag die Aufgabe zugrunde, neue verbesserte Reaktivfarbstoffe zu finden, die eine hohe Reaktivität und ein gutes Aufbauvermögen besitzen, die mit hoher Fixierausbeute gefärbt werden können, die vor allem für das Ausziehfärbeverfahren geeignet sind, und die auf cellulosehaltigem Fasermaterial nass- und lichtechte Färbungen ergeben.

Es hat sich gezeigt, dass die weiter unten definierten neuen Reaktivfarbstoffe diesen Anforderungen genügen.

Gegenstand der Erfindung sind Verbindungen der Formel

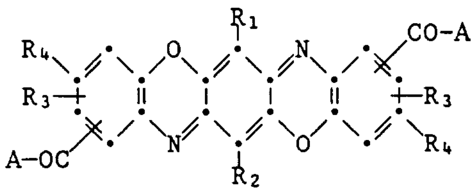

(1),

worin $R_1$, $R_2$ und $R_3$ unabhängig voneinander Wasserstoff oder ein Substituent sind,
$R_4$ für einen Rest der Formel

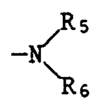

(2)

oder

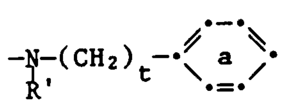

(2*),

worin $R_5$ und $R_6$ unabhängig voneinander Wasserstoff oder gegebenenfalls substituiertes $C_1$-$C_6$-Alkyl bedeuten, R' Wasserstoff oder $C_1$-$C_6$-Alkyl ist, t eine ganze Zahl von 0 bis 4 bedeutet und der Phenylrest (a) gegebenenfalls substituiert ist, steht und A ein Rest der Formel

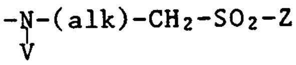

(3a),

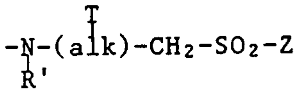

(3b),

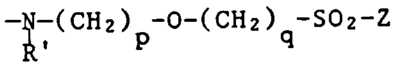

(3c),

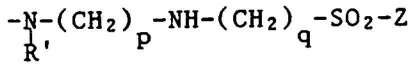

(3d),

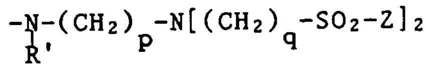

(3e)

oder

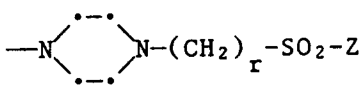

(3f),

worin R' die oben angegebene Bedeutung hat, (alk) einen $C_1$-$C_6$-Alkylenrest darstellt, T Wasserstoff, Halogen, Hydroxy, Sulfato, Carboxy, Cyano, $C_1$-$C_4$-Alkanoyloxy, $C_1$-$C_4$-Alkoxycarbonyl, Carbamoyl oder einen Rest -$SO_2$-Z bedeutet, V Wasserstoff, gegebenenfalls substituiertes $C_1$-$C_4$-Alkyl oder ein Rest der Formel -(alk)-$CH_2$-$SO_2$-Z, worin (alk) die zuvor angegebene Bedeutung hat, ist, Z einen Rest der Formel -$CH=CH_2$ oder -$CH_2$-$CH_2$-Y bedeutet, Y eine Abgangsgruppe darstellt und p, q und r unabhängig voneinander eine ganze Zahl von 1 bis 6 bedeuten, ist.

Bei den Resten $R_1$ und $R_2$ handelt es sich z.B. um Wasserstoff, Halogen wie Brom und insbesondere Chlor, $C_1$-$C_4$-Alkyl, worunter generell Methyl, Ethyl, n- oder iso-Propyl oder n-, sec- oder tert.-Butyl zu verstehen ist, $C_1$-$C_4$-Alkoxy, welches generell Methoxy, Ethoxy, n- oder iso-Propoxy oder n-, iso- oder tert.-Butoxy umfasst, Phenoxy, Carboxy, Carbamoyl oder $C_1$-$C_4$-Alkanoylamino, z.B. Acetylamino.

Vorzugsweise bedeuten $R_1$ und $R_2$ jeweils Wasserstoff, Methyl, Methoxy, Acetylamino und besonders bevorzugt Chlor.

Der Rest $R_3$ hat z.B. die Bedeutung Wasserstoff, Sulfo, $C_1$-$C_4$-Alkyl, $C_1$-$C_4$-Alkoxy, Halogen, Carboxy, Carbamoyl, N-$C_1$-$C_4$-Alkyl- oder N,N-Di-$C_1$-$C_4$-Alkylcarbamoyl, $C_1$-$C_4$-Alkylsulfonyl, Sulfamoyl oder N-$C_1$-$C_4$-Alkyl- oder N,N-Di-$C_1$-$C_4$-Alkylsulfamoyl.

Vorzugsweise handelt es sich bei $R_3$ um Wasserstoff, Chlor, $C_1$-$C_4$-Alkyl oder $C_1$-$C_4$-Alkoxy und besonders bevorzugt um Wasserstoff.

Eine bevorzugte Ausführungsform der Erfindung betrifft Verbindungen der Formel (1), worin $R_1$ und $R_2$ jeweils Chlor und $R_3$ Wasserstoff sind.

Bedeuten $R_5$ und/oder $R_6$ in Formel (2) einen gegebenenfalls substituierten $C_1$-$C_6$-Alkylrest, so handelt es sich z.B. um einen Methyl-, Ethyl-, n-oder iso-Propyl-, n-, sec.- oder tert.-Butyl- oder um einen geradkettigen oder verzweigten Pentyl- oder Hexylrest, wobei diese Reste z.B. durch Hydroxy, Sulfo, Sulfato, Carboxy, Cyano, Halogen, $C_1$-$C_4$-Alkoxycarbonyl, $C_1$-$C_4$-Alkanoyloxy oder Carbamoyl substituiert sein können und der Alkylrest mit Ausnahme von Methyl gegebenenfalls zusätzlich z.B. durch eine Gruppe -O-, -S- oder -NH- unterbrochen sein kann.

Vorzugsweise steht einer der Reste $R_5$ und $R_6$ für Wasserstoff und der andere für einen gegebenenfalls wie oben geschildert substituierten und/oder durch eine Gruppe -O-, -S- oder -NH- unterbrochenen $C_1$-$C_6$-Alkylrest.

Der Rest $R_4$ kann z.B. der Formel

R'
-N-alk-U (2a),

$-N[(alk)-U]_2$ (2b),

$-NH-(CH_2)_p-O-(CH_2)_q-U$ (2c),

$-NH-(CH_2)_p-NH-(CH_2)_q-U$ (2d) oder

U
-NH-(CH₂)-(alk)-CH₂-U (2e),

worin R', (alk), p und q die zuvor angegebene Bedeutung haben und U für Hydroxy, Sulfo, Sulfato, Carboxy, Cyano, Halogen, $C_1$-$C_4$-Alkoxycarbonyl, z.B. Methoxy- oder Ethoxycarbonyl, $C_1$-$C_4$-Alkanoylamino, besonders Acetylamino oder Propionylamino, oder Carbamoyl steht, entsprechen.

Bei R' handelt es sich z.B. um Wasserstoff, Methyl, Ethyl, n- oder iso-Propyl, n-, sec.- oder tert.-Butyl oder um einen geradkettigen oder verzweigten Pentyl- oder Hexylrest. Vorzugsweise steht R' für Wasserstoff, Methyl oder Ethyl und besonders bevorzugt für Wasserstoff.

(alk) in den Formeln (2a), (2b) und (2e) bedeutet z.B. einen Methylen-, Ethylen-, 1,3-Propylen-, 1,4-Butylen-, 1,5-Pentylen- oder 1,6-Hexylenrest oder deren verzweigte Isomere. Bevorzugt ist für (alk) die Bedeutung eines $C_1$-$C_4$-Alkylenrestes und insbesondere die Bedeutung Methylen oder Ethylen.

p und q in den Formeln (2c) und (2d) stehen unabhängig voneinander bevorzugt für eine ganze Zahl von 1 bis 4; besonders bevorzugt bedeuten p und q jeweils die Zahl 2.

U in den Formeln (2a) bis (2e) hat vorzugsweise die Bedeutung Hydroxy, Sulfo oder Sulfato und steht besonders bevorzugt für Sulfato.

Eine bevorzugte Ausführungsform der vorliegenden Erfindung betrifft Verbindungen der Formel (1), worin $R_4$ ein Rest der Formel

R''
-N-(alk')-U' (2a'),

$-N[(alk')-U']_2$ (2b'),

$-NH-CH_2-CH_2-O-CH_2-CH_2-U'$ (2c'),

$-NH-CH_2-CH_2-NH-CH_2-CH_2-U'$ (2d') oder

-NH-CH₂-(alk')-CH₂-U' (2e),
U'

worin R'' Wasserstoff, Methyl oder Ethyl, (alk') $C_1$-$C_4$-Alkylen und U Hydroxy, Sulfo oder Sulfato bedeuten, ist.

Beispiele für besonders bevorzugte Reste $R_4$ sind:

$-NH-(CH_2)_2-OSO_3H$ ,

$-NH-(CH_2)_3-OSO_3H$ ,

$-NH-(CH_2)_4-OSO_3H$ ,

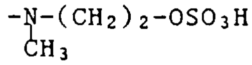 ,

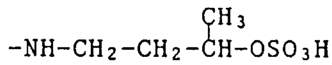 ,

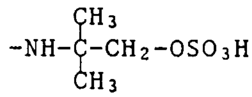 ,

-NH-$CH_2$-$CH_2$-O-$CH_2$-$CH_2$-$OSO_3H$ ,
-NH-$CH_2$-$CH_2$-NH-$CH_2$-$CH_2$-$OSO_3H$ ,

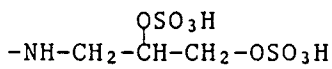 .

Bedeutet $R_4$ einen Rest der Formel (2*), so gelten für R' die zuvor angegebenen Bedeutungen und Bevorzugungen; t steht vorzugsweise für die Zahl 1 oder 2.

Geeignete Substituenten am Phenylrest (a) sind z.B. unsubstituiertes oder z.B. durch Hydroxy, Sulfo oder Sulfato substituiertes $C_1$-$C_4$-Alkyl, $C_1$-$C_4$-Alkoxy, Halogen, Sulfo, Carboxy oder Hydroxy.

Der Phenylrest (a) ist vorzugsweise unsubstituiert oder z.B durch Sulfo, Methyl und/oder Methoxy substituiert.

Stellt $R_4$ einen Rest der zuvor angegebenen Formel (2*) dar, so ist es bevorzugt, dass R' Wasserstoff und t die Zahl 1 oder 2 bedeuten und der Phenylrest (a) unsubstituiert oder durch Sulfo, Methyl und/oder Methoxy substituiert ist.

In den Formeln (3a) bis (3f) gelten für R', (alk) p und q die zuvor genannten Bedeutungen und Bevorzugungen.

T steht vorzugsweise für Wasserstoff, Hydroxy, Sulfato, Acetyloxy, Carboxy, Methoxycarbonyl, Ethoxycarbonyl oder die Gruppe -$SO_2$-Z, wobei Z die zuvor angegebene Bedeutung hat; besonders bevorzugt ist T Wasserstoff oder ein Rest -$SO_2$-Z.

Ist V ein substituierter $C_1$-$C_4$-Alkylrest, so kann dies z.B. ein durch Halogen, Hydroxy, Cyano, Carboxy, Sulfo, Sulfato, $C_1$-$C_4$-Alkoxy oder $C_1$-$C_4$-Alkoxycarbonyl substituierter $C_1$-$C_4$-Alkylrest sein.

Beispiele für substituierte $C_1$-$C_4$-Alkylreste sind: Carboxymethyl, $\beta$-Carboxyethyl, Methoxycarbonylmethyl, Ethoxycarbonylmethyl, $\beta$-Carboxyethyl, Methoxycarbonylmethyl, Ethoxycarbonylmethyl, $\beta$-Methoxyethyl, $\beta$-Ethoxyethyl, $\beta$-Chorethyl, $\gamma$-Brompropyl, $\beta$-Hydroxyethyl, $\beta$-Hydroxybutyl, $\beta$-Cyanethyl, Sulfomethyl, $\beta$-Sulfoethyl, $\beta$-Sulfatoethyl.

Steht V für einen Rest der Formel Z-$O_2$S-$H_2$C-(alk)-, so kann sich dieser von dem in Formel (3a) vorhandenen zweiten Rest Z-$O_2$S-$H_2$C-(alk)- unterscheiden oder, vorzugsweise, mit letzterem übereinstimmen.

V bedeutet vorzugweise Wasserstoff, Methyl, Ethyl oder die Gruppe Z-$O_2$S-$H_2$C(alk)-; insbesondere bevorzugt ist für V die Bedeutung Wasserstoff.

Bei Y handelt es sich z.B. um einen unter alkalischen Bedingungen abspaltbaren anorganischen oder organischen Rest.

Beispiele für geeignete Reste Y sind:
-$OSO_3H$, -$SSO_3H$, -$OCOCH_3$, -OCO-$C_6H_5$, $OPO_3H_2$, -Cl, -Br, -F,

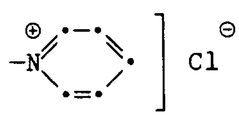 , 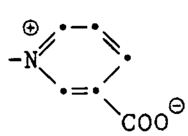 , 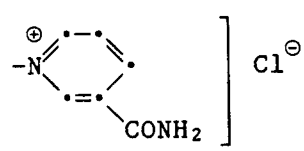 .

Vorzugsweise steht Y für die Gruppe -$OSO_3H$, -$SSO_3H$, -$OCOCH_3$, -$OPO_3H_2$ oder -Cl und besonders bevorzugt für die Gruppe -$OSO_3H$.

r in der Formel (3e) steht vorzugsweise für eine ganze Zahl von 1 bis 4 und besonders bevorzugt für die Zahl 2 oder 3.

Der Rest A entspricht vorzugsweise der Formel

$-N-(alk')-CH_2-SO_2-Z'$ (3a'),
$V'$

$SO_2-Z'$
$-NH-(alk')-CH_2-SO_2-Z'$ (3b')

$-NH-CH_2-CH_2-O-CH_2-CH_2-SO_2-Z'$ (3c')

$-NH-CH_2-CH_2-NH-CH_2-CH_2-SO_2-Z'$ (3d') oder

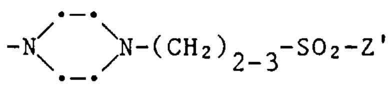
(3f'),

worin V' Wasserstoff, Methyl, Ethyl oder einen Rest $-(alk')-CH_2-SO_2-Z'$ bedeutet, (alk') ein $C_1$-$C_4$-Alkylenrest ist, Z' für Vinyl oder $-CH_2-CH_2-Y'$ steht und Y' $-OSO_3H$, $-SSO_3H$, $-OCOCH_3$, $-OPO_3H_2$ oder -Cl bedeutet.

Beispiele für besonders bevorzugte Reste A sind:
$-NH-(CH)_2-SO_2-(CH_2)_2-OSO_3H$
$-NH-(CH_2)_3-SO_2-(CH_2)_2-OSO_3H$
$-NH-(CH_2)_2-O-(CH_2)_2-SO_2-(CH_2)_2-OSO_3H$
$-N[-(CH_2)_2-SO_2-(CH_2)_2-OSO_3H]_2$

$-N-(CH_2)_2-SO_2-(CH_2)_2-OSO_3H$
$CH_3$

$-N-(CH_2)_2-SO_2-(CH_2)_2-OSO_3H$
$C_2H_5$

$SO_2-(CH_2)_2-OSO_3H$
$-NH-CH_2-CH-CH_2-CH_2-CH_2-SO_2-(CH_2)_2-OSO_3H$

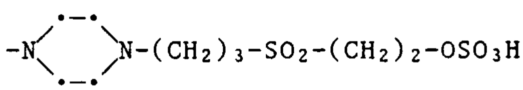

Eine bevorzugte Gruppe von erfindungsgemässen Farbstoffen umfasst Verbindungen der Formel (1), worin $R_1$ und $R_2$ jeweils Chlor bedeuten, $R_3$ Wasserstoff ist, $R_4$ einen Rest der zuvor angegebenen Formel (2a), (2b), (2c), 2d) oder (2e) bedeutet und A für einen Rest der zuvor angegebenen Formel (3a'), (3b'), (3c'), (3d') oder (3f') steht.

Besonders bevorzugt sind Reaktivfarbstoffe der Formel (1), worin $R_1$ und $R_2$ jeweils Chlor und $R_3$ Wasserstoff sind, $R_4$ einen Rest der zuvor angegebenen Formel (2a'), (2b'), (2c'), (2d') oder (2e') bedeutet und A ein Rest der Formel
$-NH-(CH_2)_2-SO_2-(CH_2)_2-OSO_3H$ ,
$-NH-(CH_2)_3-SO_2-(CH_2)_2-OSO_3H$ ,
$-NH-(CH_2)_2-O-(CH_2)_2-SO_2-(CH_2)_2-OSO_3H$ ,
$-N[(CH_2)_2-SO_2-(CH_2)_2-OSO_3H]_2$ ,

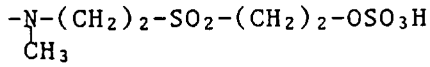

,

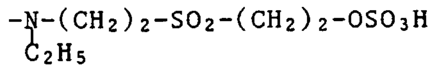

,

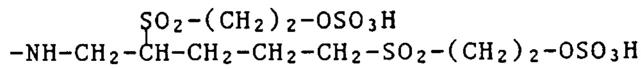

oder

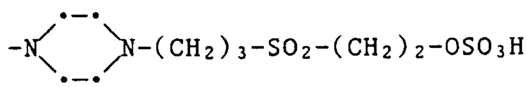

ist.

Eine weitere Gruppe von bevorzugten Reaktivfarbstoffen stellen Verbindungen der Formel (1), worin $R_1$ und $R_2$ jeweils Chlor und $R_3$ Wasserstoff sind, $R_4$ einen Rest der Formel (2*), worin R' Wasserstoff und t die Zahl 1 oder 2 bedeuten und der Phenylrest (a) unsubstituiert oder durch Sulfo, Methyl und/oder Methoxy substituiert ist, bedeutet und für A die zuvor angegebenen Bedeutungen und Bevorzugungen gelten, dar.

Die Verbindungen der Formel (1) können in an sich bekannter Weise erhalten werden, z.B. indem man ein Amin der Formel

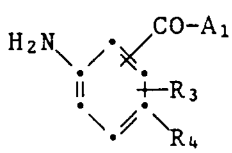

(4),

worin $R_3$ und $R_4$ die zuvor angegebene Bedeutung haben und $A_1$ die Bedeutung von A hat oder für einen Rest der zuvor angegebenen Formel (3a) bis (3f), worin Z $-CH_2-CH_2-OH$ ist, steht, mit einem 1,4-Benzochinon der Formel

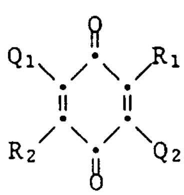

(5),

worin $R_1$ und $R_2$ die zuvor angegebene Bedeutung haben und $Q_1$ und $Q_2$ jeweils Wasserstoff, Chlor, Brom, $C_1$-$C_4$-Alkoxy oder Phenoxy bedeuten, zu einer Verbindung der Formel

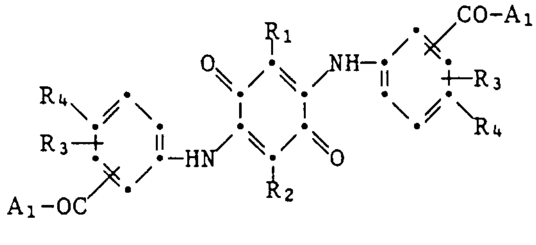

(6)

kondensiert, die erhaltene Anilverbindung durch nachfolgenden Ringschluss zur Dioxazinverbindung reagieren lässt und gegebenenfalls den Rest $A_1$ in einen Rest A umwandelt.

Die Kondensation der Amine der Formel (4) mit den Benzochinonen der Formel (5) wird vorteilhaft in einem wässrigen oder wässrig-organischen Medium unter Zugabe alkalischer Kondensationsmittel bei pH-Werten von 3 bis 11, vorzugweise 4 bis 8, und Temperaturen von 20 bis 100 °C, vorzugsweise 30 bis 60 °C oder in gepufferten Lösungen, die obige alkalische Kondensationsprodukte enthalten, durchgeführt. Man kann auch in einem rein organischen Milieu unter Zusatz säurebindender Mittel arbeiten.

Alkalische Kondensationsmittel sind z.B. Natriumhydrogencarbonat, Natriumcarbonat, Natrium- oder Kaliumacetat, Natriumhydroxid, Kaliumhydroxid, Natriumphosphate oder Natriumborat.

Die Verbindungen der Formel (4) sind neu und stellen einen weiteren Gegenstand der Erfindung dar. Sie können hergestellt werden, z.B. indem man eine Verbindung der Formel

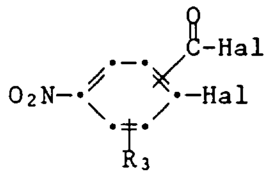 (7),

worin $R_3$ die zuvor angegebene Bedeutung hat und Hal Halogen, insbesondere Chlor, bedeutet, zunächst mit einem Amin der Formel

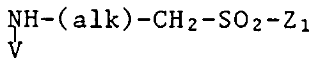 ($3a_1$),

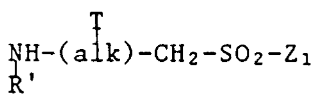 ($3b_1$),

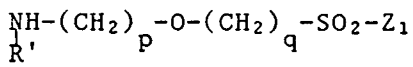 ($3c_1$),

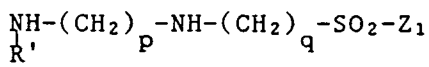 ($3d_1$),

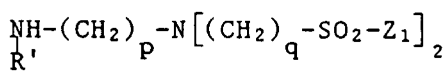 ($3e_1$)

oder

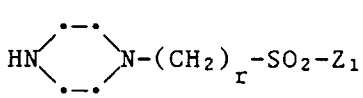 ($3f_1$),

worin (alk), V, T, R', p, q und r jeweils die zuvor angegebene Bedeutung haben und $Z_1$ die Bedeutung von Z hat oder für den Rest $-CH_2-CH_2-OH$ steht, und dann mit einem Amin der Formel

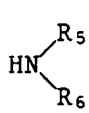 (2'),

worin $R_5$ und $R_6$ jeweils die zuvor angegebene Bedeutung haben, umsetzt, die Nitrogruppe zur Aminogruppe reduziert und gegebenenfalls den Rest $Z_1$ in einen Rest Z verwandelt, indem man eine Abgangsgruppe Y einführt.

In Abänderung des oben beschriebenen Verfahrens kann anstelle der Nitroverbindung der Formel (7) auch die entsprechende Aminoverbindung eingesetzt werden, womit der Reduktionsschritt der Nitrogruppe

entfällt. Die Nitroverbindungen der Formel (7) wie auch die entsprechenden Aminoverbindungen sind an sich bekannt oder können nach bekannten Methoden erhalten werden.

Eine weitere Variante des oben beschriebenen Verfahrens besteht darin, anstelle der Amine der Formeln ($3a_1$) bis ($3f_1$) geeignete Vorprodukte einzusetzen und diese nachträglich in die entsprechenden Amine umzuwandeln.

Geeignete Vorprodukte sind z.B. die den Formeln ($3a_1$) bis ($3f_1$) analogen Thioetheramine, die nach der Umsetzung mit einer Verbindung der Formel (7) in bekannter Weise zu den entsprechenden Sulfonen oxidiert werden können.

Es ist auch möglich, zunächst geeignete Halogenalkylamine mit dem Säurechlorid der Formel (7) reagieren zu lassen und die dabei erhaltenen Verbindungen mit 2-Mercaptoethanol und Natriumalkoholat in Alkohol zu den oben genannten Thioetheraminen umzusetzen, welche anschliessend wiederum zu den Sulfonen der Formeln ($3a_1$) bis ($3f_1$) oxidiert werden können.

Vorzugsweise werden solche Amine der Formeln ($3a_1$) bis ($3f_1$) bzw. deren Vorprodukte eingesetzt, welche eine Vorstufe des Reaktivrestes enthalten und dementsprechend $Z_1$ z.B. einen Rest der Formel $HO-H_2C-H_2C$-darstellt. Die Vorstufe des Reaktivrestes wird dann nachträglich wie weiter unten beschrieben in die Endstufe überführt.

Die Amine der Formeln ($3a_1$) bis ($3f_1$) bzw. deren Vorprodukte sind z.B. aus der EP-A 210 951 bekannt oder lassen sich analog dazu herstellen.

Die Umsetzung (Kondensation) des Säurechlorids der Formel (7) mit den vorgenannten Aminen erfolgt zum Beispiel in wässrigem, wässrigorganischem oder organischem Medium bei einer Temperatur zwischen etwa 0° und 120°C, vorzugweise 10° bis 60°C, in Gegenwart von alkalischen säurebindenden Mitteln, z.B. Alkalimetallhydroxiden, -carbonaten oder -bicarbonaten.

Falls man von Vorprodukten der Amine der Formeln ($3a_1$) bis ($3f_1$) ausgeht, kann die Oxidation der Thioetherverbindungen zu den Sulfonen nach verschiedenen Methoden erfolgen, beispielsweise mit Wasserstoffperoxid mit oder ohne Zusatz von Wolfram- oder Vanadinverbindungen als Katalysatoren, ferner mit Peressigsäure, Kaliumpermanganat oder Chromsäure, oder mit Chlor/Salzsäure je in wässrigem, wässrigorganischem oder organischem Medium.

Die Reduktion der Nitrogruppe zur Aminogruppe erfolgt in an sich bekannter Weise durch katalytische Hydrierung mit Pd/Kohlenstoff in Ethanol, Essigester oder Tetrahydrofuran bei Raumtemperatur bis etwa 40°C. Die Reduktion kann auch mit Fe/Salzsäure oder Fe/Essigsäure in wässriger Lösung durchgeführt werden.

Die so erhältlichen Carbonsäureamide, in denen die Gruppierung $-SO_2-Z_1$ eine $\beta$-Hydroxyethylsulfonylgruppe darstellt, können durch Behandeln mit Sulfatierungsmitteln, Phosphorylierungsmitteln, Halogenierungsmitteln, Alkyl- oder Arylsulfonsäurehalogeniden, Alkyl- oder Arylcarbonsäurehalogeniden oder Alkyl- oder Arylcarbonsäureanhydriden in die entsprechenden Farbstoffvorprodukte übergeführt werden, in denen die Gruppierung $-SO_2-Z_1$ z.B. die Gruppierung $-SO_2-CH_2-CH_2-O-SO_3H$, $-SO_2-CH_2-CH_2-O-PO_3H_2$, $-SO_2-CH_2-CH_2$-Halogen, $-SO_2-CH_2-CH_2-O-CO-CH_3$ oder $-SO_2-CH_2-CH_2-O-CO-C_5H_5$ bedeutet. Die so erhaltenen Produkte können ihrerseits durch Behandeln mit alkalisch wirkenden Mitteln, wie beispielsweise Alkalihydroxid oder Alkalicarbonat, wie Natriumhydroxid oder Natriumcarbonat, in entsprechende Verbindungen übergeführt werden, in denen die Gruppierung $-SO_2-Z_1$ die Gruppierung $-SO_2-CH=CH_2$ bedeutet. Die so erhaltenen Produkte können wiederum durch Umsetzung (Addition) mit Salzen der Thioschwefelsäure, wie Natriumthiosulfat, in Verbindungen übergeführt werden, in denen die Gruppierung $-SO_2-Z_1$ die Gruppierung $-SO_2-CH_2-CH_2-S-SO_3H$ bedeutet.

Geeignete Sulfatierungsmittel sind hierbei beispielsweise konzentrierte Schwefelsäure sowie Chlorsulfonsäure und Amidosulfonsäure oder andere Schwefeltrioxid abgebende Verbindungen. Geeignete Phosphorylierungsmittel sind hierbei beispielsweise konzentrierte Phosphorsäure, Pyro-, Meta- oder Polyphosphorsäure, Polyphosphorsäurealkylester, Phosphoroxichlorid oder Gemische aus Phosphorsäure und Phosphor-(V)-oxid. Als Halogenierungsmittel können beispielsweise Thionylchlorid oder Thionylbromid verwendet werden.

Weisen die Alkylreste $R_5$ und/oder $R_6$ des Aminosubstituenten $R_4$ ihrerseits Substituenten auf, so können diese schon im zugrunde liegenden Amin der Formel (2') vorhanden sein oder aber erst nach erfolgter Synthese in an sich bekannter Weise eingeführt werden. Weiterhin denkbar ist auch der nachträgliche Austausch eines im Ausgangsprodukt der Formel (2') vorhandenen Substituenten gegen einen anderen, wofür die Substitution der Hydroxygruppe durch die Sulfatogruppe mittels geeigneter schon zuvor genannter Sulfatierungsmittel ein Beispiel ist.

Die Verbindungen der Formel (2') und (5) sind bekannt oder können nach an sich bekannten Methoden erhalten werden.

Der Ringschluss der Anilverbindung der Formel (6) zur Dioxazinverbindung kann nach an sich bekannten Methoden, insbesondere in konzentrierter Schwefelsäure und vor allem in Oleum mit $SO_3$-Gehalten von 1-50 Gew.-% bei Temperaturen von 10 bis 80 °C gegebenenfalls mit Zusatz von Oxidationsmitteln wie Kalium- oder Ammoniumperoxidisulfat, Braunstein oder organischen Peroxiden, vorgenommen werden. Vorzugsweise werden in den Ausgangsprodukten vorhandene Hydroxyalkylreste, z.B. der $\beta$-Hydroxyethylsulfonylrest, unter den beim Ringschluss angewandten Reaktionsbedingungen in die entsprechenden Sulfatoalkylreste umgewandelt; in Fall des $\beta$-Sulfatoethylsulfonylrestes kann dieser gegebenenfalls nachträglich wie zuvor geschildert in die Vinylform oder in eine andere zuvor genannte Vorstufe des Vinylsulfonylrestes übergeführt werden.

Die wichtigsten Verfahrensvarianten sind in den Ausführungsbeispielen dargestellt.

Die erfindungsgemässen Farbstoffe der Formel (1) sind faserreaktiv. Unter faserreaktiven Farbstoffen sind solche zu verstehen, die mit den Hydroxylgruppen der Cellulose oder mit den reaktiven Zentren von natürlichen oder synthetischen Polyamiden unter Bildung kovalenter chemischer Bindungen zu reagieren vermögen.

Die erfindungsgemässen Reaktivfarbstoffe der Formel (1) eignen sich zum Färben und Bedrucken der verschiedensten Materialien, wie Seide, Leder, Wolle, Polyamidfasern und besonders cellulosehaltiger Fasermaterialien aller Art. Solche Fasermaterialien sind beispielsweise die natürlichen Cellulosefasern, wie Baumwolle, Leinen und Hanf, sowie Zellstoff und regenerierte Cellulose. Die erfindungsgemässen Reaktivfarbstoffe sind auch zum Färben oder Bedrucken von hydroxylgruppenhaltigen Fasern geeignet, die in Mischgeweben enthalten sind, z.B. von Gemischen aus Baumwolle mit Polyesterfasern oder Polyamidfasern.

Die erfindungsgemässen Farbstoffe lassen sich auf verschiedene Weise auf das Fasermaterial applizieren und auf der Faser fixieren, insbesondere in Form von wässrigen Farbstofflösungen und -druckpasten. Sie eignen sich sowohl für das Ausziehverfahren als auch zum Färben nach dem Foulard-Färbeverfahren, wonach die Ware mit wässrigen, gegebenenfalls salzhaltigen Farbstofflösungen imprägniert wird, und die Farbstoffe nach einer Alkalibehandlung oder in Gegenwart von Alkali, gegebenenfalls unter Wärmeeinwirkung fixiert werden. Besonders geeignet sind sie für das sogenannte Kaltverweilverfahren, wonach der Farbstoff zusammen mit dem Alkali auf dem Foulard aufgebracht wird und danach durch mehrstündiges Lagern bei Raumtemperatur fixiert wird. Nach dem Fixieren werden die Färbungen oder Drucke mit kaltem und heissem Wasser, gegebenenfalls unter Zusatz eines dispergierend wirkenden und die Diffusion der nicht fixierten Anteile fördernden Mittels gründlich gespült.

Die erfindungsgemässen Reaktivfarbstoffe zeichnen sich durch hohe Reaktivität, gutes Fixiervermögen und ein gutes Aufbauvermögen aus. Sie können daher nach dem Ausziehfärbeverfahren bei niedrigen Färbetemperaturen eingesetzt werden und erfordern beim Pad-Steam-Verfahren nur kurze Dämpfzeiten. Die Fixiergrade sind hoch, und die nicht fixierten Anteile können leicht ausgewaschen werden, wobei die Differenz zwischen Ausziehgrad und Fixiergrad bemerkenswert klein, d.h. der Seifverlust sehr gering ist. Die erfindungsgemässen Reaktivfarbstoffe eignen sich auch besonders zum Druck, vor allem auf Baumwolle, ebenso aber auch zum Bedrucken von stickstoffhaltigen Fasern, z.B. von Wolle oder Seide oder von Mischgeweben, die Wolle oder Seide enthalten.

Die mit den erfindungsgemässen Farbstoffen hergestellten Färbungen und Drucke auf Cellulosefasermaterialien besitzen eine hohe Farbstärke und eine hohe Faser-Farbstoff-Bindungsstabilität, sowohl in saurem als auch in alkalischem Bereich, weiterhin eine gute Lichtechtheit und sehr gute Nassechtheitseigenschaften, wie Wasch-, Wasser-, Seewasser-, Ueberfärbe-und Schweissechtheiten, sowie eine gute Plissierechtheit, Bügelechtheit und Reibechtheit. Hervorzuheben ist insbesondere die sehr gute Chlorechtheit der erfindungsgemässen Reaktivfarbstoffe.

Die nachfolgenden Beispiele dienen zur Erläuterung der Erfindung. Die Temperaturen sind in Celsiusgraden angegeben, Teile sind Gewichtsteile, die Prozentangaben beziehen sich auf Gewichtsprozente, sofern nicht anders vermerkt. Gewichtsteile stehen zu Volumteilen im Verhältnis von Kilogramm zu Liter.

Beispiel 1: 66,5 Teile des Amins der Formel

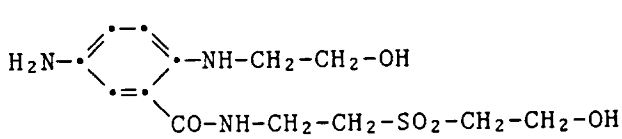

(8)

werden in 600 Teilen Wasser suspendiert und der pH-Wert der Suspension auf 6,0 eingestellt. Man gibt 24,6 Teile 2,3,5,6-Tetrachlorchinon und 80 Teile Methanol hinzu, erwärmt den Ansatz auf ca. 40 °C und hält

den pH-Wert durch Zugabe von Sodalösung bei 5,8 bis 6,0. Nach beendeter Umsetzung wird das ausgefallene Produkt abgesaugt, mit Wasser gewaschen und getrocknet. Es entspricht der Formel

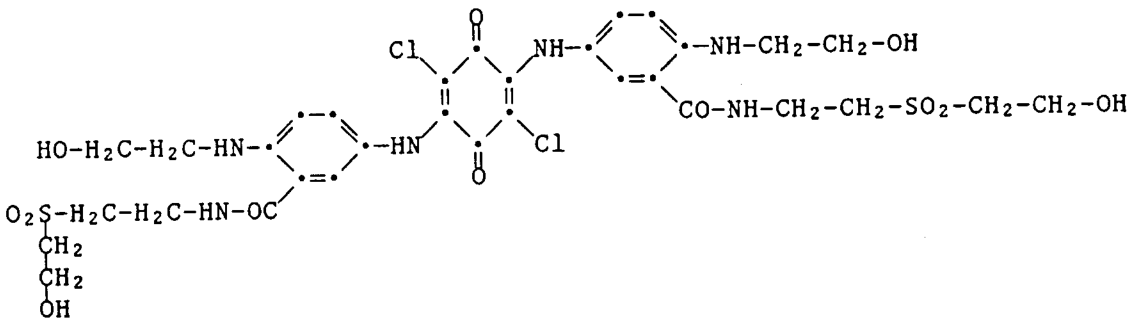

Beispiel 2: 83 Teile des gemäss Beispiel 1 erhaltenen Chloranilkondensationsproduktes werden innerhalb von etwa 1 Stunde bei 10 bis 20 °C in 200 Teile 20 %iges Oleum eingetragen. Man lässt ca. 30 bis 60 Minuten nachrühren und trägt dann 45 Teile Kaliumperoxydisulfat innerhalb von ca. 45 Minuten bei 20 bis 25 °C unter leichter Aussenkühlung in das Reaktionsgemisch ein. Nach einem 1-stündigen Nachrühren wird das Reaktionsgemisch in 1500 Teile Eis gegeben und die dabei entstehende klare blaue Lösung zunächst mit 7,5 Teilen Trinatriumphosphat, dann mit 4 n Natriumhydroxidlösung unter Rühren und Kühlen versetzt, bis ein pH-Wert von 4,5 bis 5,0 erreicht worden ist. Der ausgefallene Farbstoff wird abgesaugt, gewaschen und im Vakuum getrocknet; er entspricht der Formel

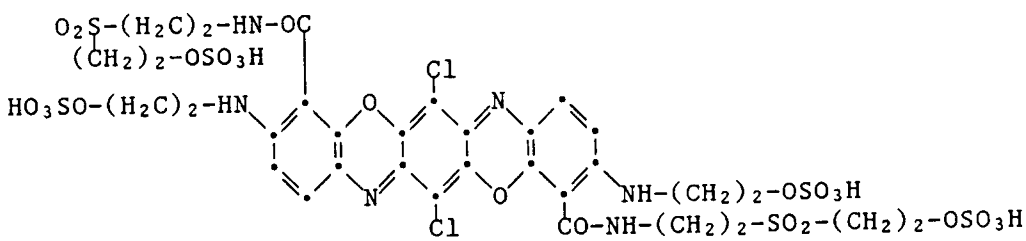

und färbt Baumwolle in kräftigen Blautönen mit ausgezeichneten Allgemeinechtheiten.

Beispiel 3 bis 31: Verfährt man wie in den Beispielen 1 und 2 beschrieben, verwendet jedoch anstelle des Amins der Formel (8) äquivalente Mengen der in der folgenden Tabelle angegebenen Amine, werden analoge Farbstoffe erhalten, die jeweils Baumwolle in einer blauen Nuance mit guten Allgemeinechtheiten färben.

| Beispiel Nr. | Amin |
|---|---|
| 3 | 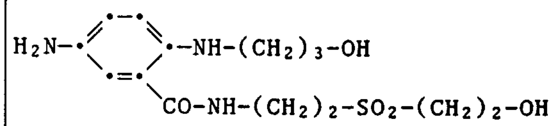 |
| 4 | 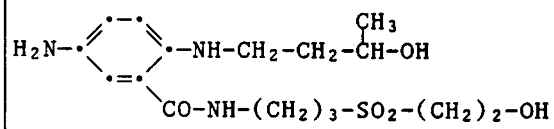 |
| 5 | 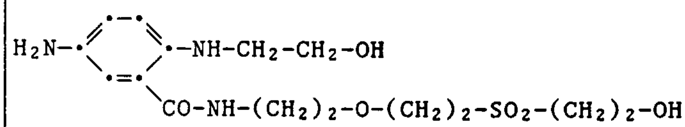 |
| 6 | 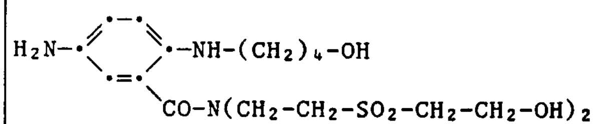 |
| 7 | 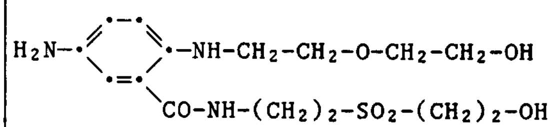 |
| 8 | 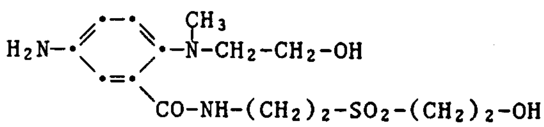 |
| 9 | 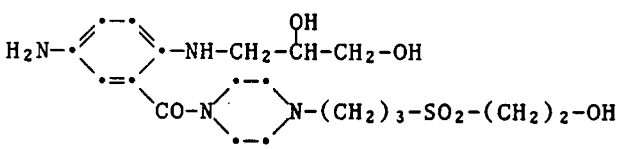 |
| 10 | 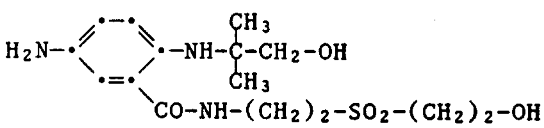 |
| 11 | 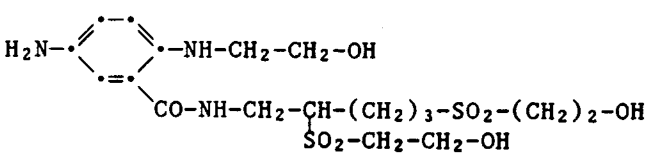 |

| Beispiel Nr. | Amin |
|---|---|
| 12 | 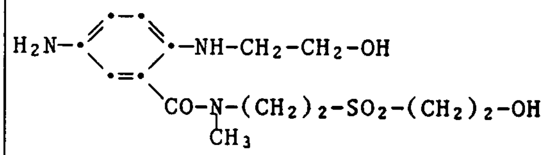 |
| 13 | 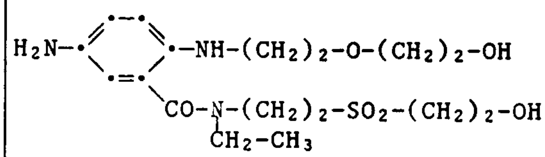 |
| 14 | 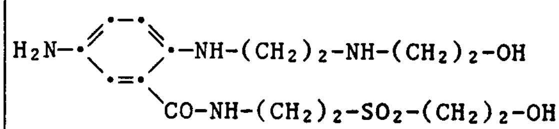 |
| 15 | 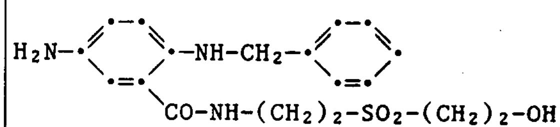 |
| 16 | 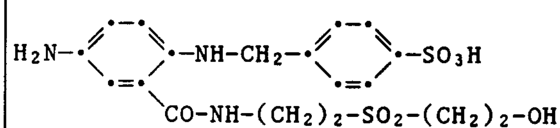 |
| 17 | 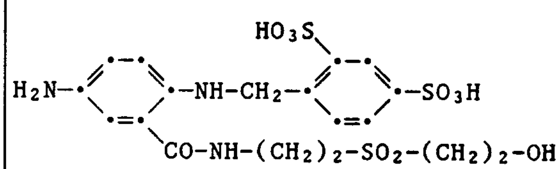 |
| 18 | 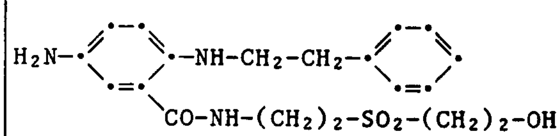 |
| 19 | 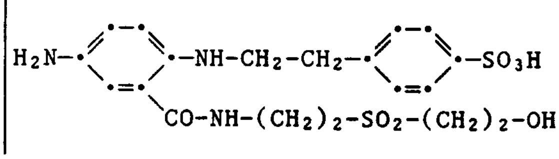 |

| Beispiel Nr. | Amin |
|---|---|
| 20 | 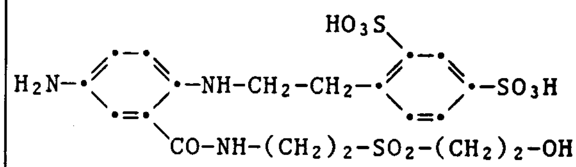 |
| 21 | 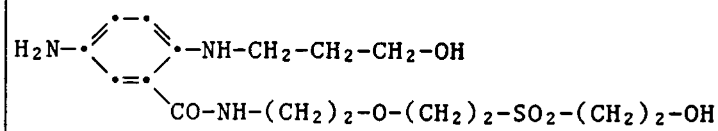 |
| 22 | 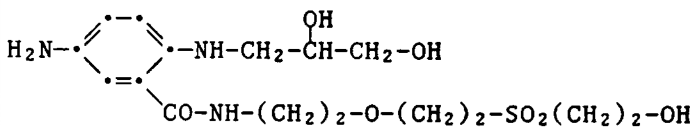 |
| 23 | 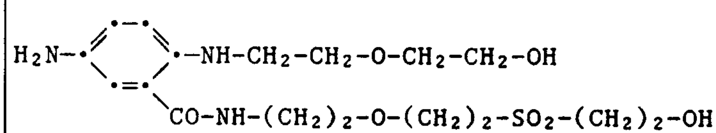 |
| 24 | 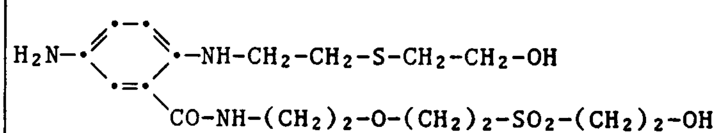 |
| 25 | 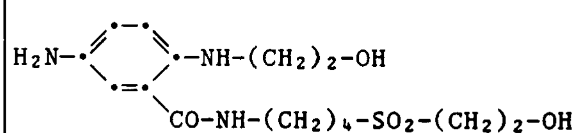 |
| 26 | 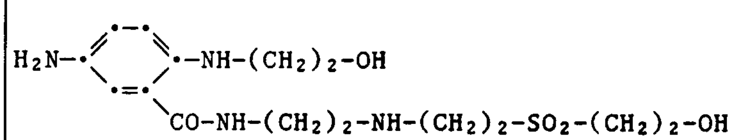 |
| 27 | 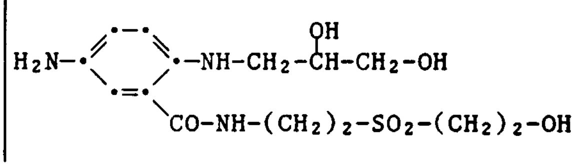 |

| Beispiel Nr. | Amin |
|---|---|
| 28 | 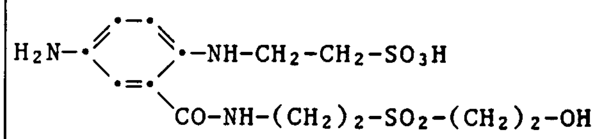 |
| 29 | 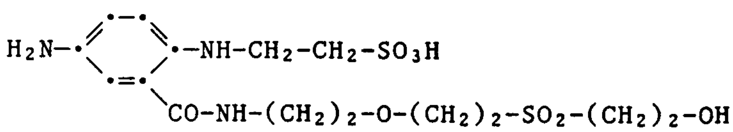 |
| 30 | 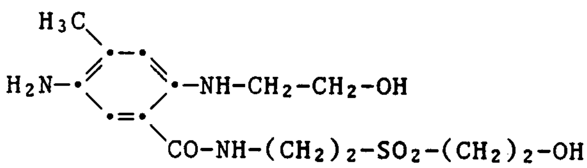 |
| 31 | 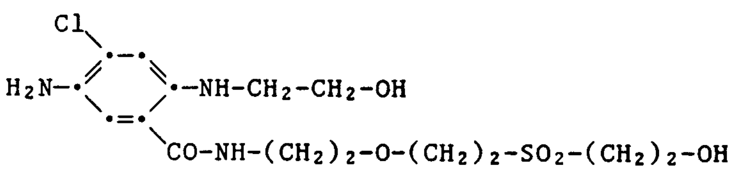 |

Färbevorschrift I

2 Teile des gemäss Beispiel 1 erhaltenen Farbstoffes werden in 400 Teilen Wasser gelöst; dazu gibt man 1500 Teile einer Lösung, die pro Liter 53 g Natriumchlorid enthält. In dieses Färbebad geht man bei 40°C mit 100 Teilen eines Baumwollgewebes ein. Nach 45 Minuten werden 100 Teile einer Lösung, die pro Liter 16 g Natriumhydroxid und 20 g kalzinierte Soda enthält, zugegeben. Die Temperatur des Färbebades wird weitere 45 Minuten bei 40°C gehalten. Danach wird die gefärbte Ware gespült, während einer Viertelstunde mit einem nichtionogenen Waschmittel kochend geseift, nochmals gespült und getrocknet.

Färbevorschrift II

2 Teile des gemäss Beispiel 1 erhaltenen Reaktivfarbstoffes werden in 400 Teilen Wasser gelöst; dazu gibt man 1500 Teile einer Lösung, die pro Liter 53 g Natriumchlorid enthält. In dieses Färbebad geht man bei 35°C mit 100 Teilen eines Baumwollgewebes ein. Nach 20 Minuten werden 100 Teile einer Lösung, die pro Liter 16 g Natriumhydroxid und 20 g kalzinierte Soda enthält, zugegeben. Die Temperatur des Färbebades wird weitere 15 Minuten bei 35°C gehalten. Danach wird die Temperatur innerhalb von 20 Minuten von 60°C erhöht. Die Temperatur wird weitere 35 Minuten bei 60°C gehalten. Danach wird gespült, während einer Viertelstunde mit einem nichtionogenen Waschmittel kochend geseift, nochmals gespült und getrocknet.

Färbevorschrift III

8 Teile des gemäss Beispiel 1 erhaltenen Reaktivfarbstoffes werden in 400 Teilen Wasser gelöst; dazu gibt man 1400 Teile einer Lösung, die pro Liter 100 g Natriumsulfat enthält. In dieses Färbebad geht man bei 25°C mit 100 Teilen eines Baumwollgewebes ein. Nach 10 Minuten werden 200 Teile einer Lösung, die pro Liter 150 g Trinatriumphosphat enthält, zugegeben. Danach wird die Temperatur des Färbebades

innerhalb 10 Minuten auf 60 °C erhöht. Die Temperatur wird weitere 90 Minuten auf 60 °C gehalten. Danach wird gespült, während einer Viertelstunde mit einem nichtionogenen Waschmittel kochend geseift, nochmals gespült und getrocknet.

Färbevorschrift IV

4 Teile des gemäss Beispiel 1 erhaltenen Reaktivfarbstoffes werden in 50 Teilen Wasser gelöst. Dazu gibt man 50 Teile einer Lösung, die pro Liter 5 g Natriumhydroxid und 20 g kalzinierte Soda enthält. Mit der erhaltenen Lösung wird ein Baumwollgewebe foulardiert, so dass es um 70 % seines Gewichtes zunimmt, und dann auf eine Kaule aufgewickelt. Das Baumwollgewebe wird so während 3 Stunden bei Raumtemperatur gelagert. Danach wird die gefärbte Ware gespült, während einer Viertelstunde mit einem nichtionogenen Waschmittel kochend geseift, nochmals gespült und getrocknet.

Färbevorschrift V

6 Teile des gemäss Beispiel 1 erhaltenen Reaktivfarbstoffes werden in 50 Teilen Wasser gelöst. Dazu gibt man 50 Teile einer Lösung, die pro Liter 16 g Natriumhydroxid und 0,04 Liter Wasserglas (38 ° Bé) enthält. Mit der erhaltenen Lösung wird ein Baumwollgewebe foulardiert, so dass es um 70 % seines Gewichtes zunimmt, und dann auf eine Kaule aufgewickelt. Das Baumwollgewebe wird so während 10 Stunden bei Raumtemperatur gelagert. Danach wird die gefärbte Ware gespült, während einer Viertelstunde mit einem nichtionogenen Waschmittel kochend geseift, nochmals gespült und getrocknet.

Färbevorschrift VI

2 Teile des gemäss Beispiel 1 erhaltenen Reaktivfarbstoffes werden unter Zusatz von 0,5 Teilen m-nitrobenzolsulfonsaurem Natrium in 100 Teilen Wasser gelöst. Mit der erhaltenen Lösung wird ein Baumwollgewebe imprägniert, so dass es um 75 % seines Gewichtes zunimmt, und dann getrocknet. Dann imprägniert man das Gewebe mit einer 20 °C warmen Lösung, die pro Liter 4 g Natriumhydroxid und 300 g Natriumchlorid enthält, quetscht auf 75 % Gewichtszunahme ab, dämpft die Färbung während 30 Sekunden bei 100 bis 102 °C, spült, seift während einer Viertelstunde in einer 0,3%igen kochenden Lösung eines nichtionogenen Waschmittels, spült und trocknet.

Druckvorschrift I

3 Teile des gemäss Beispiel 1 erhaltenen Reaktivfarbstoffes werden unter schnellem Rühren in 100 Teile einer Stammverdickung, enthaltend 50 Teile 5%ige Natriumalginatverdickung, 27,8 Teile Wasser, 20 Teile Harnstoff, 1 Teil m-nitrobenzolsulfonsaures Natrium sowie 1,2 Teile Natriumhydrogencarbonat, eingestreut. Mit der so erhaltenen Druckpaste bedruckt man ein Baumwollgewebe, trocknet und dämpft den erhaltenen bedruckten Stoff 2 Minuten bei 102 °C in gesättigtem Dampf. Das bedruckte Gewebe wird dann gespült, gegebenenfalls kochend geseift und nochmals gespült, und anschliessend getrocknet.

Druckvorschrift II

5 Teile des gemäss Beispiel 1 erhaltenen Reaktivfarbstoffes werden unter schnellem Rühren in 100 Teile einer Stammverdickung, enthaltend 50 Teile 5%ige Natriumalginatverdickung, 36,5 Teile Wasser, 10 Teile Harnstoff, 1 Teil m-nitrobenzolsulfonsaures Natrium sowie 2,5 Teile Natriumhydrogencarbonat, eingestreut. Mit der so erhaltenen Druckpaste, deren Stabilität den technischen Anforderungen entspricht, bedruckt man ein Baumwollgewebe, trocknet und dämpft den erhaltenen bedruckten Stoff 8 Minuten bei 102 °C in gesättigtem Dampf. Das bedruckte Gewebe wird dann gespült, gegebenenfalls kochend geseift und nochmals gespült, und anschliessend getrocknet.

## Patentansprüche

1. Verbindungen der Formel

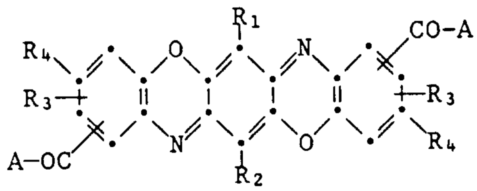

(1),

worin $R_1$, $R_2$ und $R_3$ unabhängig voneinander Wasserstoff oder ein Substituent sind, $R_4$ für einen Rest der Formel

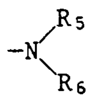

(2)

oder

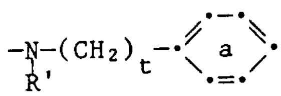

(2*),

worin $R_5$ und $R_6$ unabhängig voneinander Wasserstoff oder gegebenenfalls substituiertes $C_1$-$C_6$-Alkyl bedeuten, R' Wasserstoff oder $C_1$-$C_6$-Alkyl ist, t eine ganze Zahl von 0 bis 4 bedeutet und der Phenylrest (a) gegebenenfalls substituiert ist, steht und A ein Rest der Formel

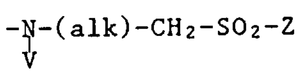

(3a),

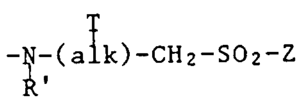

(3b),

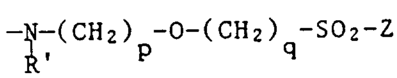

(3c),

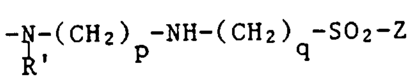

(3d),

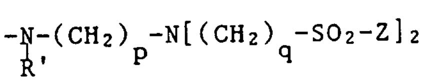

(3e)

oder

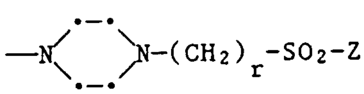

(3f),

worin R' die oben angegebene Bedeutung hat, (alk) einen $C_1$-$C_6$-Alkylenrest darstellt, T Wasserstoff, Halogen, Hydroxy, Sulfato, Carboxy, Cyano, $C_1$-$C_4$-Alkanoyloxy, $C_1$-$C_4$-Alkoxycarbonyl, Carbamoyl oder einen Rest $-SO_2$-Z bedeutet, V Wasserstoff, gegebenenfalls substituiertes $C_1$-$C_4$-Alkyl oder ein Rest der Formel -(alk)-$CH_2$-$SO_2$-Z, worin (alk) die zuvor angegebene Bedeutung hat, ist, Z einen Rest der Formel $-CH=CH_2$ oder $-CH_2$-$CH_2$-Y bedeutet, Y eine Abgangsgruppe darstellt und p, q und r unabhängig voneinander eine ganze Zahl von 1 bis 6 bedeuten, ist.

2. Verbindungen gemäss Anspruch 1, dadurch gekennzeichnet, dass $R_1$ und $R_2$ unabhängig voneinander Wasserstoff, Halogen, $C_1$-$C_4$-Alkyl, $C_1$-$C_4$-Alkoxy, Phenoxy, Carboxy, Carbamoyl oder $C_1$-$C_4$-Alkanoylamino sind und $R_3$ Wasserstoff, Sulfo, $C_1$-$C_4$-Alkyl, $C_1$-$C_4$-Alkoxy, Halogen, Carboxy, Carbamoyl, N-$C_1$-$C_4$-Alkyl- oder N,N-Di-$C_1$-$C_4$-Alkylcarbamoyl, $C_1$-$C_4$-Alkylsulfonyl, Sulfamoyl oder N-$C_1$-$C_4$-Alkyl- oder N,N-Di-$C_1$-$C_4$-Alkylsulfamoyl bedeutet.

3. Verbindungen gemäss Anspruch 1 oder 2, dadurch gekennzeichnet, dass $R_1$ und $R_2$ jeweils Wasserstoff, Methyl, Methoxy, Acetylamino oder Chlor, sind und $R_3$ Wasserstoff, Chlor, $C_1$-$C_4$-Alkyl oder $C_1$-$C_4$-Alkoxy bedeutet.

4. Verbindungen gemäss Anspruch 3, dadurch gekennzeichnet, dass $R_1$ und $R_2$ jeweils Chlor und $R_3$ Wasserstoff sind.

5. Verbindungen gemäss einem der Ansprüche 1 bis 4, dadurch gekennzeichnet, dass $R_4$ für einen Rest der im Anspruch 1 angegebenen Formel (2) steht und $R_5$ und $R_6$ unabhängig voneinander Wasserstoff oder unsubstituiertes oder durch Hydroxy, Sulfo, Sulfato, Carboxy, Cyano, Halogen, $C_1$-$C_4$-Alkoxycarbonyl, $C_1$-$C_4$-Alkanoyloxy oder Carbamoyl substituiertes und mit Ausnahme von Methyl gegebenenfalls durch -O-, -S- oder -NH- unterbrochenes $C_1$-$C_6$-Alkyl bedeuten.

6. Verbindungen gemäss einem der Ansprüche 1 bis 5, dadurch gekennzeichnet, dass es sich bei $R_4$ um einen Rest der Formel

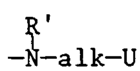 (2a),

-N[(alk)-U]$_2$ (2b),

-NH-$(CH_2)_p$-O-$(CH_2)_q$-U (2c),

-NH-$(CH_2)_p$-NH-$(CH_2)_q$-U (2d) oder

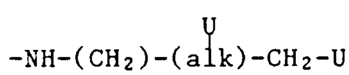 (2e),

worin R', (alk), p und q die im Anspruch 1 angegebene Bedeutung haben und U für Hydroxy, Sulfo, Sulfato, Carboxy, Cyano, Halogen, $C_1$-$C_4$-Alkoxycarbonyl, $C_1$-$C_4$-Alkanoylamino, oder Carbamoyl steht, handelt.

7. Verbindungen gemäss einem der Ansprüche 1 bis 6, dadurch gekennzeichnet, dass $R_4$ ein Rest der Formel

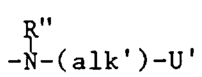 (2a'),

-N[(alk')-U']$_2$ (2b'),

$-NH-CH_2-CH_2-O-CH_2-CH_2-U'$ (2c'),

$-NH-CH_2-CH_2-NH-CH_2-CH_2-U'$ (2d') oder

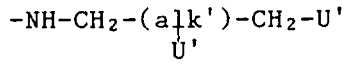 (2e),

worin R'' Wasserstoff, Methyl oder Ethyl, (alk') $C_1$-$C_4$-Alkylen und U Hydroxy, Sulfo oder Sulfato bedeuten, ist.

**8.** Verbindungen gemäss einem der Ansprüche 1 bis 4, dadurch gekennzeichnet, dass $R_4$ für einen Rest der im Anspruch 1 angegebenen Formel (2*) steht, R' Wasserstoff und t die Zahl 1 oder 2 bedeuten und der Phenylrest (a) unsubstituiert oder durch Sulfo, Methyl und/oder Methoxy substituiert ist.

**9.** Verbindungen gemäss einem der Ansprüche 1 bis 8, dadurch gekennzeichnet, dass es sich bei der Abgangsgruppe Y um einen Rest $-OSO_3H$, $-SSO_3H$, $-OCOCH_3$, $-OCO-C_6H_5$, $OPO_3H_2$, -Cl, -Br, -F,

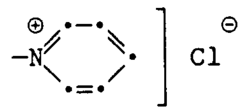 , 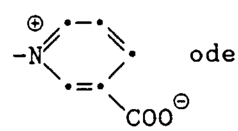 oder 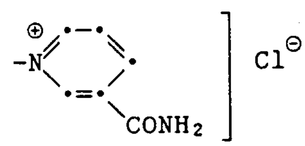 .

handelt.

**10.** Verbindungen gemäss einem der Ansprüche 1 bis 9, dadurch gekennzeichnet, dass es sich bei der Abgangsgruppe Y um einen Rest $-OSO_3H$, $-SSO_3H$, $-OCOCH_3$, $-OPO_3H_2$ oder -Cl, vorzugsweise $-OSO_3H$, handelt.

**11.** Verbindungen gemäss einem der Ansprüche 1 bis 10, dadurch gekennzeichnet, dass A ein Rest der Formel

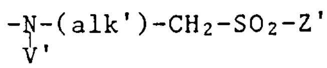 (3a'),

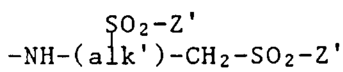 (3b')

$-NH-CH_2-CH_2-O-CH_2-CH_2-SO_2-Z'$ (3c')

$-NH-CH_2-CH_2-NH-CH_2-CH_2-SO_2-Z'$ (3d') oder

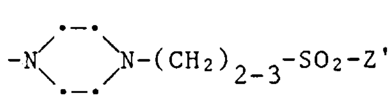 (3f'),

worin V' Wasserstoff, Methyl, Ethyl oder einen Rest $-(alk')-CH_2-SO_2-Z'$ bedeutet, (alk') ein $C_1$-$C_4$-Alkylenrest ist, Z' für Vinyl oder $-CH_2-CH_2-Y'$ steht und Y' $-OSO_3H$, $-SSO_3H$, $-OCOCH_3$, $-OPO_3H_2$ oder -Cl bedeutet, ist.

**12.** Verbindungen gemäss einem der Ansprüche 1 bis 11, dadurch gekennzeichnet, dass A ein Rest der Formel
$-NH-(CH)_2-SO_2-(CH_2)_2-OSO_3H$ ,

$-NH-(CH_2)_3-SO_2-(CH_2)_2-OSO_3H$ ,
$-NH-(CH_2)_2-O-(CH_2)_2-SO_2-(CH_2)_2-OSO_3H$ ,
$-N[(CH_2)_2-SO_2-(CH_2)_2-OSO_3H]_2$ ,

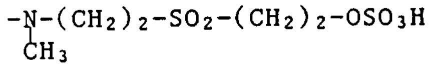 ,

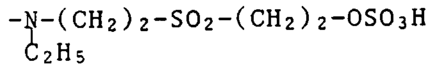 ,

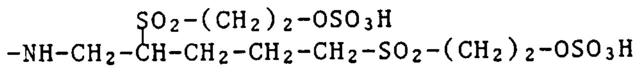

oder

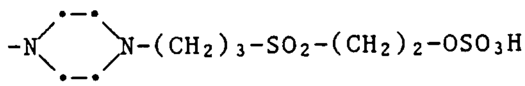

ist.

**13.** Verbindungen der Formel (1) gemäss Anspruch 1, worin $R_1$ und $R_2$ jeweils Chlor bedeuten, $R_3$ Wasserstoff ist, $R_4$ einen Rest der im Anspruch 6 angegebenen Formel (2a), (2b), (2c), (2d) oder (2e) bedeutet und A für einen Rest der im Anspruch 11 angegebenen Formel (3a'), (3b'), (3c'), (3d') oder (3f') steht.

**14.** Verbindungen der Formel (1) gemäss Anspruch 1, worin $R_1$ und $R_2$ jeweils Chlor und $R_3$ Wasserstoff sind, $R_4$ einen Rest der im Anspruch 7 angegebenen Formel (2a'), (2b'), (2c'), (2d') oder (2e') bedeutet und A ein Rest der Formel
$-NH-(CH_2)_2-SO_2-(CH_2)_2-OSO_3H$ ,
$-NH-(CH_2)_3-SO_2-(CH_2)_2-OSO_3H$ ,
$-NH-(CH_2)_2-O-(CH_2)_2-SO_2-(CH_2)_2-OSO_3H$ ,
$-N[(CH_2)_2-SO_2-(CH_2)_2-OSO_3H]_2$ ,

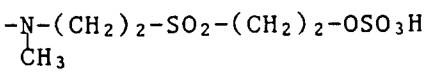 ,

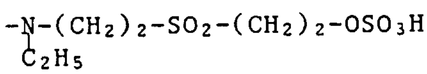 ,

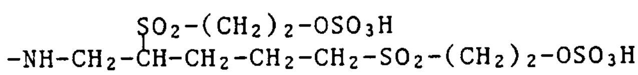

oder

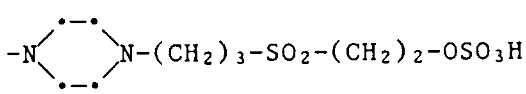

ist.

**15.** Verbindungen der Formel (1) gemäss Anspruch 1, worin $R_1$ und $R_2$ jeweils Chlor und $R_3$ Wasserstoff sind, $R_4$ für einen Rest der Formel (2*), worin R' Wasserstoff und t die Zahl 1 oder 2 bedeuten und der Phenylrest (a) unsubstituiert oder durch Sulfo, Methyl und/oder Methoxy substituiert ist, steht und A die in Anspruch 14 angegebene Bedeutung hat.

**16.** Verfahren zur Herstellung von Verbindungen der Formel (1), dadurch gekennzeichnet, dass man ein Amin der Formel

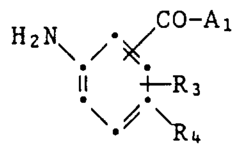

(4),

worin $R_3$ und $R_4$ die im Anspruch 1 angegebene Bedeutung haben und $A_1$ die im Anspruch 1 angegebene Bedeutung von A hat oder für einen Rest der im Anspruch 1 angegebenen Formel (3a) bis (3e), worin Z $-CH_2-CH_2-OH$ ist, steht, mit einem 1,4-Benzochinon der Formel

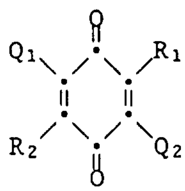

(5),

worin $R_1$ und $R_2$ die im Anspruch 1 angegebene Bedeutung haben und $Q_1$ und $Q_2$ jeweils Wasserstoff, Chlor, Brom, $C_1$-$C_4$-Alkoxy oder Phenoxy bedeuten, zu einer Verbindung der Formel

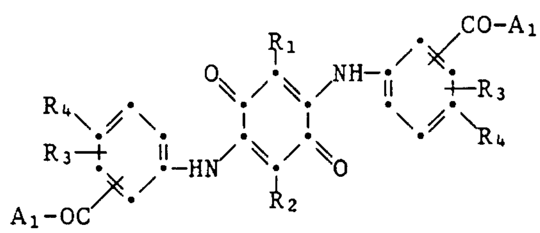

(6)

kondensiert, die erhaltene Anilverbindung durch nachfolgenden Ringschluss zur Dioxazinverbindung reagieren lässt und gegebenenfalls den Rest $A_1$ in einen Rest A umwandelt.

**17.** Verwendung der Verbindungen der Formel (1) als Reaktivfarbstoffe zum Färben und Bedrucken von cellulosehaltigen Fasermaterialien.

**18.** Verwendung gemäss Anspruch 17, zum Färben und Bedrucken von Baumwolle.

**19.** Verbindungen der Formel

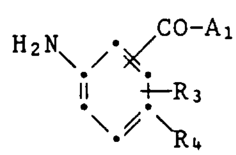

(4),

worin $R_3$ und $R_4$ die im Anspruch 1 angegebene Bedeutung haben und $A_1$ die im Anspruch 1 angegebene Bedeutung von A hat oder für einen Rest der im Anspruch 1 angegebenen Formel (3a) bis (3f), worin Z $-CH_2-CH_2-OH$ ist, steht.

## Claims

**1.** A compound of the formula

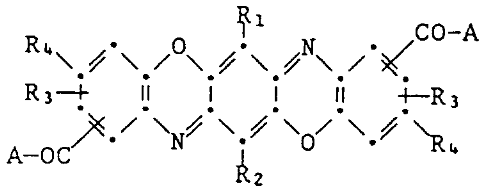 (1),

where $R_1$, $R_2$ and $R_3$ are each independently of the others hydrogen or a substituent, $R_4$ is a radical of the formula

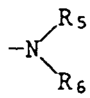 (2)

or

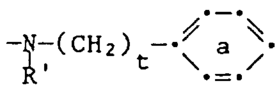 (2*),

where $R_5$ and $R_6$ are each independently of the other hydrogen or substituted or unsubstituted $C_1$-$C_6$alkyl, R' is hydrogen or $C_1$-$C_6$alkyl, t is an integer from 0 to 4 and the phenyl radical (a) may be substituted, and A is a radical of the formula

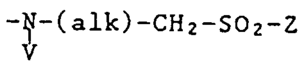 (3a),

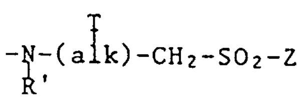 (3b),

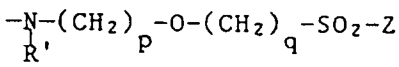 (3c),

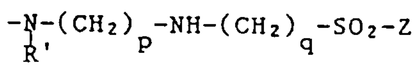 (3d),

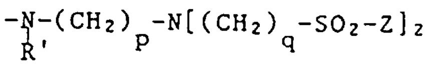 (3e)

or

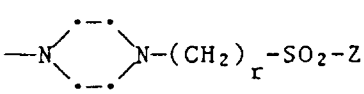 (3f),

where R' is as defined above, (alk) is a $C_1$-$C_6$alkylene radical, T is hydrogen, halogen, hydroxyl, sulfato,

carboxyl, cyano, $C_1$-$C_4$alkanoyloxy, $C_1$-$C_4$alkoxycarbonyl, carbamoyl or a radical -$SO_2$-Z, V is hydrogen, substituted or unsubstituted $C_1$-$C_4$alkyl or a radical of the formula -(alk)-$CH_2$-$SO_2$-Z, where (alk) is as defined above, Z is a radical of the formula -CH=$CH_2$ or -$CH_2$-$CH_2$-Y, Y is a leaving group, and p, q and r are each independently of the others an integer from 1 to 6.

**2.** A compound according to claim 1, wherein $R_1$ and $R_2$ are each independently of the other hydrogen, halogen, $C_1$-$C_4$alkyl, $C_1$-$C_4$alkoxy, phenoxy, carboxyl, carbamoyl or $C_1$-$C_4$alkanoylamino and $R_3$ is hydrogen, sulfo, $C_1$-$C_4$alkyl, $C_1$-$C_4$alkoxy, halogen, carboxyl, carbamoyl, $C_1$-$C_4$alkylcarbamoyl, N,N-di-$C_1$-$C_4$alkylcarbamoyl, N-$C_1$-$C_4$alkylsulfonyl, sulfamoyl, $C_1$-$C_4$alkylsulfamoyl or N,N-di-$C_1$-$C_4$alkylsulfamoyl.

**3.** A compound according to claim 1 or 2, wherein $R_1$ and $R_2$ are each hydrogen, methyl, methoxy, acetylamino or chlorine and $R_3$ is hydrogen, chlorine, $C_1$-$C_4$alkyl or $C_1$-$C_4$alkoxy.

**4.** A compound according to claim 3, wherein $R_1$ and $R_2$ are each chlorine and $R_3$ is hydrogen.

**5.** A compound according to any one of claims 1 to 4, wherein $R_4$ is a radical of the formula (2) indicated in claim 1 and $R_5$ and $R_6$ are independently of each other hydrogen or unsubstituted or hydroxyl-, sulfo-, sulfato-, carboxyl-, cyano-, halogen-, $C_1$-$C_4$alkoxycarbonyl-, $C_1$-$C_4$alkanoyloxy- or carbamoyl-substituted and uninterrupted or, except in the case of methyl, -O-, -S-, or -NH-interrupted $C_1$-$C_6$alkyl.

**6.** A compound according to any one of claims 1 to 5, wherein $R_4$ is a radical of the formula

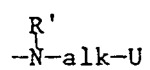 (2a),

-N[(alk)-U]$_2$ (2b),

-NH-$(CH_2)_p$-O-$(CH_2)_q$-U (2c),

-NH-$(CH_2)_p$-NH-$(CH_2)_q$-U (2d) or

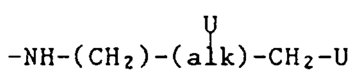 (2e),

where R', (alk), p and q are as defined in claim 1 and U is hydroxyl, sulfo, sulfato, carboxyl, cyano, halogen, $C_1$-$C_4$alkoxycarbonyl, $C_1$-$C_4$alkanoylamino or carbamoyl.

**7.** A compound according to any one of claims 1 to 6, wherein $R_4$ is a radical of the formula

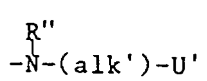 (2a'),

-N[(alk')-U']$_2$ (2b'),

-NH-$CH_2$-$CH_2$-O-$CH_2$-$CH_2$-U' (2c'),

-NH-$CH_2$-$CH_2$-NH-$CH_2$-$CH_2$-U' (2d') or

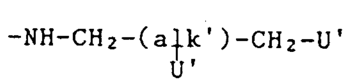 (2e'),

where R'' is hydrogen, methyl or ethyl, (alk') is $C_1$-$C_4$alkylene and U' is hydroxyl, sulfo or sulfato.

**8.** A compound according to any one of claims 1 to 4, wherein $R_4$ is a radical of the formula (2*) indicated in claim 1, R' is hydrogen, t is 1 or 2 and the phenyl radical (a) is unsubstituted or sulfo-, methyl- and/or methoxy-substituted.

**9.** A compound according to any one of claims 1 to 8, wherein the leaving group Y is a radical $-OSO_3H$, $-SSO_3H$, $-OCOCH_3$, $-OCO-C_6H_5$, $OPO_3H_2$, -Cl, -Br, -F,

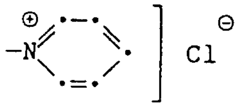 , 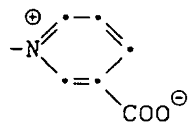 or 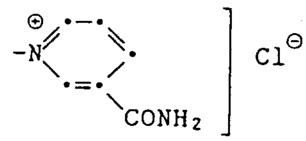 .

**10.** A compound according to any one of claims 1 to 9, wherein the leaving group Y is a radical $-OSO_3H$, $-SSO_3H$, $-OCOCH_3$, $-OPO_3H_2$ or -Cl, preferably $-OSO_3H$.

**11.** A compound according to any one of claims 1 to 10, wherein A is a radical of the formula

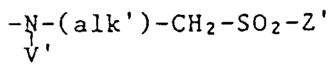 (3a'),

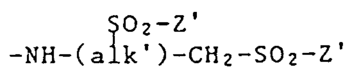 (3b')

$-NH-CH_2-CH_2-O-CH_2-CH_2-SO_2-Z'$ (3c')

$-NH-CH_2-CH_2-NH-CH_2-CH_2-SO_2-Z'$ (3d') or

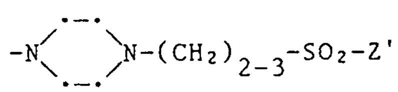 (3f'),

where V' is hydrogen, methyl, ethyl or a radical $-(alk')-CH_2-SO_2-Z'$, (alk') is $C_1$-$C_4$alkylene, Z' is vinyl or $-CH_2-CH_2-Y'$, and Y' is $-OSO_3H$, $-SSO_3H$, $-OCOCH_3$, $-OPO_3H_2$ or -Cl.

**12.** A compound according to any one of claims 1 to 11, wherein A is a radical of the formula $-N[(CH_2)_2-SO_2-(CH_2)_2-OSO_3H]_2$ ,

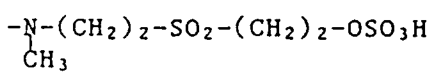 ,

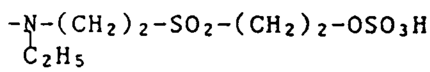 ,

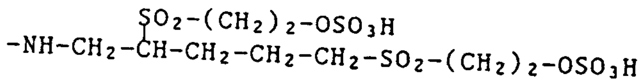

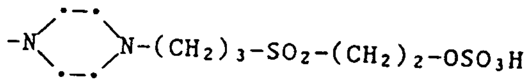

**13.** A compound of the formula (1) according to claim 1, wherein $R_1$ and $R_2$ are each chlorine, $R_3$ is hydrogen, $R_4$ is a radical of the formula (2a), (2b), (2c), (2d) or (2e) indicated in claim 6 and A is a radical of the formula (3a'), (3b'), (3c'), (3d') or (3f') indicated in claim 11.

**14.** A compound of the formula (1) according to claim 1, wherein $R_1$ and $R_2$ are each chlorine, $R_3$ is hydrogen, $R_4$ is a radical of the formula (2a'), (2b'), (2c'), (2d') or (2e') indicated in claim 7 and A is a radical of the formula

$-NH-(CH_2)_2-SO_2-(CH_2)_2-OSO_3H$ ,
$-NH-(CH_2)_3-SO_2-(CH_2)_2-OSO_3H$ ,
$-NH-(CH_2)_2-O-(CH_2)_2-SO_2-(CH_2)_2-OSO_3H$ ,
$-N[(CH_2)_2-SO_2-(CH_2)_2-OSO_3H]_2$ ,

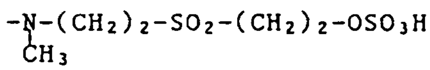 ,

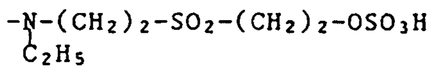 ,

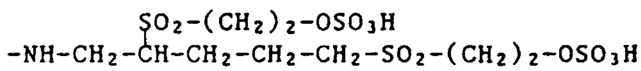

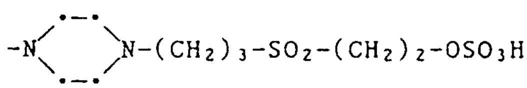

**15.** A compound of the formula (1) according to claim 1, wherein $R_1$ and $R_2$ are each chlorine, $R_3$ is hydrogen, $R_4$ is a radical of the formula (2*), where R' is hydrogen, t is 1 or 2, the phenyl radical (a) is unsubstituted or sulfo-, methyl- and/or methoxy-substituted, and A is as defined in claim 14.

**16.** A process for preparing a compound of the formula (1), which comprises condensing an amine of the formula

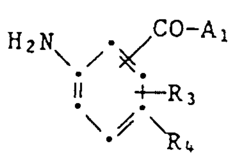 (4),

where $R_3$ and $R_4$ are as defined in claim 1 and $A_1$ is defined in the same way as A in claim 1 or is a radical of the formula (3a) to (3e) indicated in claim 1, where Z is $-CH_2-CH_2-OH$, with a 1,4-benzoquinone of the formula

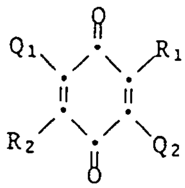

(5),

where $R_1$ and $R_2$ are as defined in claim 1 and $Q_1$ and $Q_2$ are each hydrogen, chlorine, bromine, $C_1$-$C_4$alkoxy or phenoxy, to give a compound of the formula

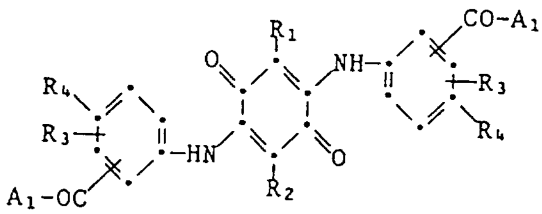

(6)

reacting the anil compound obtained by subsequent ring closure to form a dioxazine compound, and if necessary converting the radical $A_1$ into a radical A.

**17.** The use of a compound of the formula (1) as a reactive dye for dyeing and printing cellulose-containing fibre materials.

**18.** The use according to claim 17 for dyeing and printing cotton.

**19.** A compound of the formula

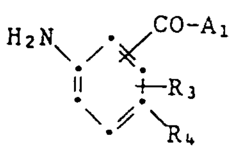

(4),

where $R_3$ and $R_4$ are as defined in claim 1 and $A_1$ is defined in the same way as A in claim 1 or is a radical of the formula (3a) to (3f) indicated in claim 1, where Z is $-CH_2-CH_2-OH$.

**Revendications**

**1.** Composés de formule

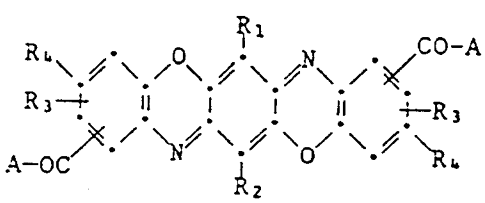

(1),

dans laquelle $R_1$, $R_2$ et $R_3$, indépendamment l'un de l'autre, représentent un atome d'hydrogène ou un substituant, $R_4$ représente un reste de formule

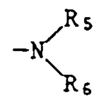
(2)

ou

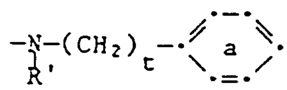
(2*),

dans laquelle $R_5$ et $R_6$ représentent, indépendamment l'un de l'autre, un atome d'hydrogène ou un groupe alkyle en $C_{1-6}$ éventuellement substitué, R' représente un atome d'hydrogène ou un groupe alkyle en $C_{1-6}$, t représente un nombre entier valant de 0 à 4, et le reste phényle (a) est éventuellement substitué,
et A représente un reste de formule

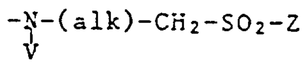
(3a),

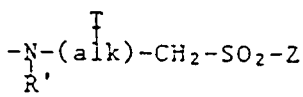
(3b),

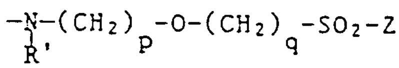
(3c),

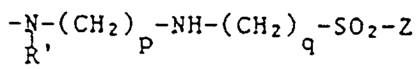
(3d),

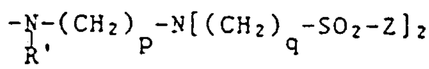
(3e)

ou

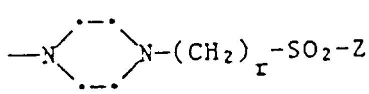
(3f),

formules dans lesquelles R' a la signification indiquée ci-dessus, (alk) représente un reste alkylène en $C_{1-6}$, T représente un atome d'hydrogène ou d'halogène ou un groupe hydroxy, sulfato, carboxy, cyano, alcanoyloxy en $C_{1-4}$, (alcoxy en $C_{1-4}$)-carbonyle ou carbamyle, ou encore un reste de formule $-SO_2-Z$, V représente un atome d'hydrogène, un groupe alkyle en $C_{1-4}$ éventuellement substitué ou un reste de formule $-(alk)-CH_2-SO_2-Z$, (alk) ayant la signification indiquée plus haut, Z représente un reste de formule $-CH=CH_2$ ou $-CH_2-CH_2-Y$, Y représente un groupe partant et p, q et r, indépendamment l'un de l'autre, représentent un nombre entier valant de 1 à 6.

2. Composés conformes à la revendication 1, **caractérisés** en ce que $R_1$ et $R_2$ représentent chacun, indépendamment l'un de l'autre, un atome d'hydrogène ou d'halogène ou un groupe alkyle en $C_{1-4}$, alcoxy en $C_{1-4}$, phénoxy, carboxy, carbamyle ou alcanoylamino en $C_{1-4}$, et $R_3$ représente un atome d'hydrogène ou d'halogène ou un groupe sulfo, alkyle en $C_{1-4}$, alcoxy en $C_{1-4}$, carboxy, carbamyle, N-(alkyle en $C_{1-4}$)-carbamyle, N,N-di-(alkyle en $C_{1-4}$)-carbamyle, alkylsulfonyle en $C_{1-4}$, sulfamyle, N-(alkyle en $C_{1-4}$)-sulfamyle ou N,N-di-(alkyle en $C_{1-4}$)-sulfamyle.

**3.** Composés conformes à la revendication 1 ou 2, **caractérisés** en ce que $R_1$ et $R_2$ représentent chacun un atome d'hydrogène ou de chlore ou un groupe méthyle, méthoxy ou acétylamino, et $R_3$ représente un atome d'hydrogène ou de chlore ou un groupe alkyle en $C_{1-4}$ ou alcoxy en $C_{1-4}$.

**4.** Composés conformes à la revendication 3, **caractérisés** en ce que $R_1$ et $R_2$ représentent chacun un atome de chlore et $R_3$ représente un atome d'hydrogène.

**5.** Composés conformes à l'une des revendications 1 à 4, **caractérisés** en ce que $R_4$ représente un groupe de formule (2) indiquée dans la revendication 1, et $R_5$ et $R_6$, indépendamment l'un de l'autre, représentent un atome d'hydrogène ou un groupe alkyle en $C_{1-6}$, non-substitué ou substitué par hydroxy, sulfo, sulfato, carboxy, cyano, halogéno, (alcoxy en $C_{1-4}$)-carbonyle, alcanoyloxy en $C_{1-4}$ ou carbamyle, et éventuellement interrompu, à l'exception du groupe méthyle, par -O-, -S- ou -NH-.

**6.** Composés conformes à l'une des revendications 1 à 5, **caractérisés** en ce que $R_4$ représente un reste de formule

$$\begin{array}{c} R' \\ | \\ -N-alk-U \end{array} \qquad (2a),$$

-N[(alk)-U)$_2$ (2b),

-NH-$(CH_2)_p$-O-$(CH_2)_q$-U (2c),

-NH-$(CH_2)_p$-NH-$(CH_2)_q$-U (2d), ou

$$-NH-(CH_2)-(\overset{\displaystyle U}{\overset{|}{alk}})-CH_2-U \qquad (2e),$$

formules dans lesquelles R', (alk), p et q ont les significations indiquées dans la revendication 1 et U représente un atome d'halogène ou un groupe hydroxy, sulfo, sulfato, carboxy, cyano, (alcoxy en $C_{1-4}$)-carbonyle, alcanoylamino en $C_{1-4}$ ou carbamyle.

**7.** Composés conformes à l'une des revendications 1 à 6, **caractérisés** en ce que $R_4$ représente un groupe de formule

$$\begin{array}{c} R'' \\ | \\ -N-(alk')-U' \end{array} \qquad (2a'),$$

-N[(alk')-U']$_2$ (2b'),

-NH-$CH_2$-$CH_2$-O-$CH_2$-$CH_2$-U' (2c'),

-NH-$CH_2$-$CH_2$-NH-$CH_2$-$CH_2$-U' (2d'), ou

$$-NH-CH_2-(\underset{\displaystyle U'}{\underset{|}{alk'}})-CH_2-U' \qquad (2e),$$

formules dans lesquelles R'' représente un atome d'hydrogène ou un groupe méthyle ou éthyle, (alk') représente un groupe alkylène en $C_{1-4}$ et U représente un groupe hydroxy, sulfo ou sulfato.

**8.** Composés conformes à l'une des revendications 1 à 4, **caractérisés** en ce que $R_4$ représente un reste de formule (2*) indiquée dans la revendication 1, R' représente un atome d'hydrogène et t

représente le nombre 1 ou 2, et le reste phényle (a) n'est pas substitué ou est substitué par sulfo, méthyle et/ou méthoxy.

**9.** Composés conformes à l'une des revendications 1 à 8, **caractérisés** en ce que, en ce qui concerne le groupe partant Y, il s'agit d'un reste $-OS_3H$, $-SSO_3H$, $-OCOCH_3$, $-OCO-C_6H_5$, $OPO_3H_2$, -Cl, -Br, -F,

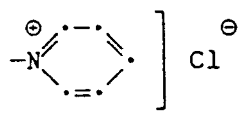 , 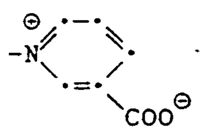 ou 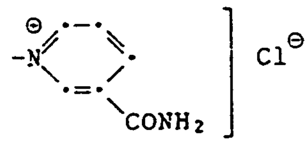 .

**10.** Composés conformes à l'une des revendications 1 à 9, **caractérisés** en ce que, en ce qui concerne le groupe partant Y, il s'agit d'un reste $-OSO_3H$, $-SSO_3H$, $-OCOCH_3$, $-OPO_3H_2$ ou -Cl, et de préférence $-OSO_3H$.

**11.** Composés conformes à l'une des revendications 1 à 10, **caractérisés** en ce que A représente un reste de formule

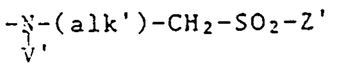 (3a'),

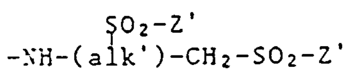 (3b')

$-NH-CH_2-CH_2-O-CH_2-CH_2-SO_2-Z'$ (3c')

$-NH-CH_2-CH_2-NH-CH_2-CH_2-SO_2-Z'$ (3d') ou

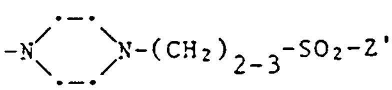 (3f'),

formules dans lesquelles V' représente un atome d'hydrogène ou un groupe méthyle ou éthyle ou encore un reste de formule $-(alk')-CH_2-SO_2-Z'$, (alk') représente un reste alkylène en $C_{1-4}$, Z' représente un groupe vinyle ou $-CH_2-CH_2-Y'$, et Y' représente $-OSO_3H$, $-SSO_3H$, $-OCOCH_3$, $-OPO_3H_2$ ou -Cl.

**12.** Composés conformes à l'une des revendications 1 à 11, **caractérisés** en ce que A représente un reste de formule

$-NH-(CH)_2-SO_2-(CH_2)_2-OSO_3H$ ,

$-NH-(CH_2)_3-SO_2-(CH_2)_2-OSO_3H$ ,

$-NH-(CH_2)_2-O-(CH_2)_2-SO_2-(CH_2)_2-OSO_3H$ ,

$-N[(CH_2)_2-SO_2-(CH_2)_2-OSO_3H]_2$ ,

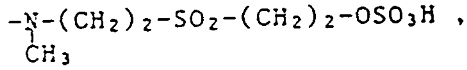

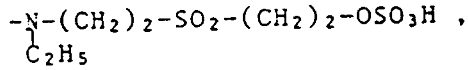

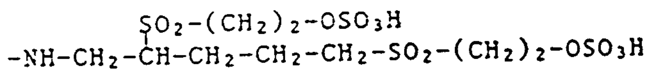

ou

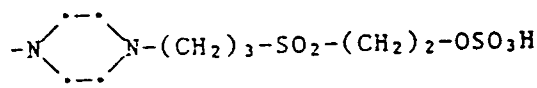

ist.

**13.** Composés de formule (1) conformes à la revendication 1, dans lesquels $R_1$ et $R_2$ représentent chacun un atome de chlore, $R_3$ représente un atome d'hydrogène, $R_4$ représente un reste de formule (2a), (2b), (2c), (2d) ou (2e) indiquée dans la revendication 6, et A représente un reste de formules (3a'), (3b'), (3c'), (3d') ou (3f') indiquée dans la revendication 11.

**14.** Composés de formule (1) conformes à la revendication 1, dans lesquels $R_1$ et $R_2$ représentent chacun un atome de chlore et $R_3$ représente un atome d'hydrogène, $R_4$ représente un reste de formule ((2a'), (2b'), (2c'), (2d') ou (2e') indiquée dans la revendication 7, et A représente un reste de formule

$-NH-(CH_2)_2-SO_2-(CH_2)_2-OSO_3H$ ,

$-NH-(CH_2)_3-SO_2-(CH_2)_2-OSO_3H$ ,

$-NH-(CH_2)_2-O-(CH_2)_2-SO_2-(CH_2)_2-OSO_3H$ ,

$-N[(CH_2)_2-SO_2-(CH_2)_2-OSO_3H]_2$ ,

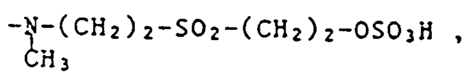

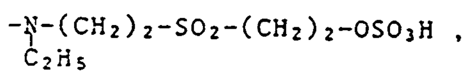

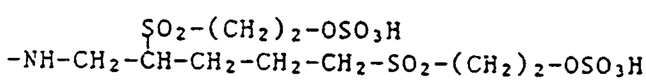

ou

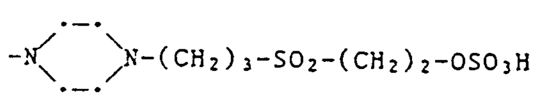

**15.** Composés de formule (1) conformes à la revendication 1, dans lesquels $R_1$ et $R_2$ représentent chacun un atome de chlore et $R_3$ représente un atome d'hydrogène, $R_4$ représente un reste de formule (2*) dans laquelle R' représente un atome d'hydrogène et t représente le nombre 1 ou 2, le reste phényle (a) étant non-substitué ou substitué par sulfo, méthyle et/ou méthoxy, et A a la signification indiquée dans la revendication 14.

**16.** Procédé de préparation de composés de formule (1), **caractérisé** en ce que l'on condense une amine de formule

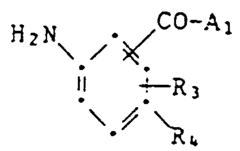

(4)

dans laquelle $R_3$ et $R_4$ ont les significations indiquées dans la revendication 1 et $A_1$ a la signification de A indiquée dans la revendication 1, ou représente un reste de l'une des formules (3a) à (3e) indiquées dans la revendication 1, où Z représente $-CH_2-CH_2-OH$, avec une 1,4-benzoquinone de formule

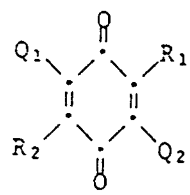

(5)

dans laquelle $R_1$ et $R_2$ ont les significations indiquées dans la revendication 1 et $Q_1$ et $Q_2$ représentent chacun un atome d'hydrogène, de chlore ou de brome ou un groupe alcoxy en $C_{1-4}$ ou phénoxy, en un composé de formule

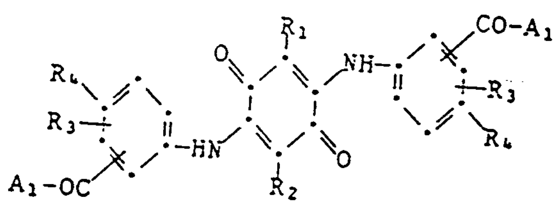

(6),

on fait réagir le composé de type anile obtenu pour le transformer en un composé de type dioxazine, par fermeture ultérieure de cycle, et l'on transforme éventuellement le reste $A_1$ en un reste A.

**17.** Utilisation des composés de formule (1) comme colorants réactifs pour la teinture et l'impression de matériaux fibreux contenant de la cellulose.

**18.** Utilisation conforme à la revendication 17, pour la teinture et l'impression du coton.

**19.** Composés de formule

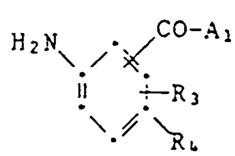

(4),

dans laquelle $R_3$ et $R_4$ ont les significations indiquées dans la revendication 1 et $A_1$ a la signification de A indiquée dans la revendication 1 ou représente un reste de l'une des formules (3a) à (3f) indiquées dans la revendication 1, où Z représente $-CH_2-CH_2-OH$.